# EUROPEAN PATENT APPLICATION

(11) **EP 4 516 925 A1**
(43) Date of publication of application: **05.03.2025**
(21) Application number: 24186547.6
(22) Date of filing: 04.07.2024
(51) Int. Cl.: C12Q 1/6851

(54) **DNA DETECTION METHOD AND DNA DETECTION KIT UNDER SPECIAL CONSIDERATION OF FLUORESCENCE INTENSITY MEASUREMENT AND MELTING CURVE ANALYSIS**

(30) Priority: 31.08.2023 JP 2023140686
(71) Applicant: Hitachi, Ltd., Tokyo 100-8280 (JP)
(72) Inventor: TANAKA, Junko, Japan, 100-8280 (JP); NAKAGAWA, Tatsuo, Japan, 100-8280 (JP)
(74) Representative: MERH-IP Matias Erny Reichl Hoffmann Patentanwälte PartG mbB

(57) **Abstract**

The present invention relates to a DNA detection method and a DNA detection kit for analyzing a gene, and more specifically, to a DNA detection method and a DNA detection kit for detecting a gene using a fluorescent-labeled probe.

## Description

### CROSS-REFERENCE TO RELATED APPLICATION

This application claims priority to JP Patent Application No. 2023-140686 filed on August 31, 2023, the content of which is incorporated herein by reference in its entirety.

### Technical Field

The present invention relates to a DNA detection method and a DNA detection kit for analyzing a gene, and more specifically, to a DNA detection method and a DNA detection kit for detecting a gene using a fluorescent-labeled probe.

### Background Art

Known genetic tests include techniques using polymerase chain reaction (PCR), real-time PCR, digital PCR, and the like. In the real-time PCR and digital PCR that enable quantification of DNA with higher accuracy than the PCR, DNA is detected using an intercalator or a fluorescent-labeled probe. Two types: a hydrolysis probe and a molecular beacon are often used in the DNA detection method using a fluorescent-labeled probe. The hydrolysis probe is degraded through the nuclease activity of DNA polymerase, the fluorescent dye is dissociated from the probe, thereby emitting fluorescence. Unlike the hydrolysis probe, the molecular beacon has a characteristic of not being degraded during PCR, the molecular beacon forms a stem-loop when it is present in a free form and binds to the loop portion of DNA to be detected. Thus, it is possible to perform not only the determination whether the amplification can be carried out based on the fluorescence intensity, but also the melting curve analysis after PCR.

Here, in DNA detection using a molecular beacon, there has been reported a method of asymmetrically amplifying DNA to be detected by making concentrations of a forward primer and a reverse primer asymmetric such that a strand complementary to a molecular beacon is excessively amplified, in order to increase the amount of binding between the molecular beacon and the amplified DNA to be detected and increase the fluorescence intensity (NPL 1).

The present inventors have developed a technique of applying an asymmetric PCR using a molecular beacon to digital PCR, and identifying the genotype of a target DNA with high sensitivity and high multiplex by melting curve analysis after amplification (PTL 1 and NPL 2).

An example of a detection method of digital PCR is shown below. First, a DNA polymerase, a primer set, and a fluorescent-labeled probe necessary for PCR are added to a limitedly diluted specimen to prepare a PCR reaction solution. The PCR reaction solution is divided into micro partitions such as wells or droplets. At this time, one molecule of the target DNA is contained or not contained in each partition. Next, the target DNA in the micro partition is amplified by PCR. The target DNA can be quantified by measuring a fluorescence intensity of each micro partition after PCR and counting the number of micro partitions having a fluorescence intensity exceeding threshold.

When the asymmetric PCR using a molecular beacon is applied, melting curve analysis is performed on the target DNA amplified in the micro partition and the molecular beacon after PCR, and a genotype of the target DNA can be identified by a difference in melting temperature (Tm).

### Citation List

### Patent Literature

PTL 1: US 2019/0352699 A1

### Non Patent Literatures

NPL 1: BMC Microbiol., 13: 295, 2013
NPL 2: Anal. Chem., 92: 11705-11713, 2020

### Summary of Invention

### Technical Problem

The present inventors have recognized for the first time that the magnitude of variation in melting temperature in the measurement data (distribution of melting temperatures measured when the target DNA is present or when the target DNA is not present) varies depending on the type of the target DNA and the sequence of the molecular beacon (Example 1). Upon identifying a plurality of types of target genes or target DNAs using molecular beacons having different sequences, the beacons being modified with an identical fluorescent dye, based on the melting temperature, a large variation in melting temperature causes overlapping of distributions of melting temperatures of different target genes or target DNAs. As a result, based on the values of the measured melting temperatures, a target DNA may be erroneously determined as a gene different from the target DNA, and a false positive may occur.

Therefore, an object of the present invention is to provide a DNA detection method and a DNA detection kit which can reduce the magnitude of variation in melting temperature of a target DNA to be identified based on a difference in melting temperatures, and accurately determine and quantify the target DNA.

### Solution to Problem

The present inventors have compared the melting curve of a gene having a large variation in melting temperature with the melting curve of a gene having a small variation in melting temperature, and found that the melting curve of the gene having a small variation in melting temperature has a large change in the fluorescence intensity accompanying the temperature change, and thus the magnitude of the peak of the differential curve of the melting curve is large. Further, the present inventors have revealed that, in a combination of a gene and a molecular beacon in which the variation in melting temperature is small, when the sequences of the gene and the molecular beacon are observed, the ratio at which a molecular beacon binding region of one strand of DNA excessively amplified by the asymmetric PCR forms a double-stranded structure is small. Therefore, the present inventors have found that when an additive for preventing secondary structure formation of a nucleic acid such as DNA is added to a reaction solution, the reaction solution is divided into micro partitions, and PCR and melting curve analysis are performed in each of the micro partitions, resulting an increased change in the fluorescence intensity accompanying the temperature change of the melting curve, and a reduction in the variation in melting temperature, and thus completed the present invention.

According to one aspect of the present invention, there is provided
a DNA detection method including the steps of:
mixing a fluorescent-labeled probe for a target DNA, a primer set for amplifying a region containing the target DNA, a test biological sample, an enzyme, and an additive for preventing DNA secondary structure formation to prepare a reaction solution;
dividing the reaction solution into a plurality of micro partitions and performing an amplification reaction in each of the plurality of micro partitions;
measuring a fluorescence intensity for each of the plurality of micro partitions while changing a temperature and measuring binding between the DNA amplified in the amplification reaction and the probe;
performing melting curve analysis on each of the micro partitions based on the measurement result to calculate a melting temperature; and/or
determining the presence or absence and/or the type of the target DNA from the fluorescence color, fluorescence intensity, and melting temperature for each of the micro partitions.

In another aspect, the present invention provides
a DNA detection kit including:
a primer pair for amplifying a region including a target DNA, the primer pair including a forward primer and a reverse primer;
a fluorescent-labeled probe for the target DNA, which binds to DNA amplified using the forward primer and the reverse primer; and/or
an additive for preventing DNA secondary structure formation.

### Advantageous Effects of Invention

According to the present invention, there are provided a DNA detection method and a DNA detection kit which can accurately and sensitively detect or quantify a target DNA, particularly a plurality of types of target genes or target DNAs, based on a difference in melting temperatures obtained by melting curve analysis. Therefore, the present invention is useful in the fields of basic research, test, drug discovery, and the like for detecting genes, identifying genotypes, and the like.

### Brief Description of Drawings

FIG. 1 is a schematic view showing an example of a method for measuring a melting temperature of DNA using a fluorescent-labeled probe.
FIG. 2 shows graphs showing an example of a result obtained by a method for determining and counting the presence or absence of a plurality of genes in each micro partition based on the melting temperature measured using a fluorescent-labeled probe in digital PCR (dPCR).
FIG. 3 shows graphs showing an example of melting curves in a case where a gene having a large variation in melting temperature (A and B) and a gene having a small variation in melting temperature (C to F) are measured.
FIG. 4 is a flowchart showing one embodiment of a DNA detection method including: adding an additive for preventing DNA secondary structure formation to a reaction solution; performing digital PCR using melting curve analysis; and quantifying the copy number of target DNA.
FIG. 5 shows graphs showing measurement results in a case where digital PCR is performed without adding an additive for preventing DNA secondary structure formation, and melting curve analysis is performed in Example 1.
FIG. 6A is a view showing the predicted secondary structure and probe binding region of an amplicon of a KRAS gene having a small variation in melting temperature.
FIG. 6B is a view showing the predicted secondary structure and probe binding region of an amplicon of a hTERT gene having a large variation in melting temperature.
FIG. 6C is a view showing the predicted secondary structure and probe binding region of an amplicon of GNAS gene having a large variation in melting temperature.
FIGS. 7A and 7B are graphs showing measurement results in a case where betaine (trimethylglycine) is added as the additive for preventing DNA secondary structure formation in real-time PCR, and melting curve analysis is performed in Example 2.
FIGS. 7C and 7D are graphs showing measurement results in a case where DMSO is added as the additive for preventing DNA secondary structure formation in real-time PCR, and melting curve analysis is performed in Example 2.
FIGS. 7E and 7F are graphs showing measurement results in a case where formamide is added as the additive for preventing DNA secondary structure formation in real-time PCR, and melting curve analysis is performed in Example 2.
FIG. 8 shows graphs showing measurement results in a case where an additive for preventing DNA secondary structure formation is added in digital PCR, and melting curve analysis of hTERT gene is performed in Example 3.
FIG. 9 shows graphs showing measurement results in a case where an additive for preventing DNA secondary structure formation is added in digital PCR, and melting curve analysis of GNAS gene is performed in Example 3.
FIG. 10 shows graphs showing measurement results regarding the relationship between the concentration of trimethylglycine and the variation in melting temperature in a case where melting curve analysis is performed by adding trimethylglycine: a type of betaine in digital PCR in Example 3. A represents the hTERT gene, and B represents the GNAS gene.

### Description of Embodiments

Objects, characteristics, advantages, and ideas thereof of the present invention are apparent to those skilled in the art from the description of the present specification. Those skilled in the art can easily reproduce the present invention from the description herein. Embodiments of the present invention, specific examples thereof, and so forth, which are shown below, indicate preferred embodiments of the present invention and are described for description and explanation, and the present invention is not limited thereto. It will be apparent to those skilled in the art that various alterations and modifications can be made based on the description herein without departing from the spirit and scope of the present invention disclosed herein.

### (1) DNA Detection Method

In one aspect, the present invention provides a DNA detection method, and the method includes the steps of:
mixing a fluorescent-labeled probe for a target DNA, a primer set for amplifying a region containing the target DNA, a test biological sample, an enzyme, and an additive for preventing DNA secondary structure formation to prepare a reaction solution;
dividing the reaction solution into a plurality of micro partitions and performing an amplification reaction in each of the plurality of micro partitions;
measuring a fluorescence intensity for each of the plurality of micro partitions while changing a temperature and measuring binding between the DNA amplified in the amplification reaction and the probe;
performing melting curve analysis on each of the micro partitions based on the measurement result to calculate a melting temperature; and
determining the presence or absence and/or the type of the target DNA from the fluorescence color, fluorescence intensity, and melting temperature of each of the micro partitions.

According to the present invention, the test biological sample may not be particularly limited as long as it is a biological sample that contains DNA containing or possibly containing a target DNA to be detected, and examples thereof include body fluid samples (such as blood and urine), tissues, and cultured cells, may include DNAs extracted and purified from these samples (such as genomic DNA and circulating DNA), or may include DNAs derived from mRNA extracted and purified from the samples (such as cDNA). Alternatively, synthesized DNA may be used as a test biological sample to design a suitable probe and/or primer. When the test biological sample is a body fluid sample, a tissue, a cultured cell, or the like, it may be preferable to make DNA or the like available by pretreatment in advance for the amplification reaction performed by the method of the present invention. Methods for preparing DNA from such samples are known in the art, and kits for simply purifying DNA, and kits for extracting mRNA and synthesizing cDNA are also commercially available.

According to the present invention, the target DNA may be a site or region of specific DNA to be detected, and may include, for example, a gene, a mutation in a gene, a regulatory sequence of a gene, and a mutation in a regulatory sequence of a gene. The target DNA may be one type or a plurality of types.

According to the method of the present invention, the reaction solution may be prepared by mixing a fluorescent-labeled probe for a target DNA, a primer set for amplifying a region containing the target DNA, a test biological sample, an enzyme, and an additive for preventing DNA secondary structure formation.

The probe may be designed such that at least a part thereof has a sequence specific to the nucleotide sequence of the target DNA, i.e., a sequence complementary to the nucleotide sequence of the gene. In a case where a plurality of target genes or target DNAs are to be detected, probes are required for each of the plurality of target genes or target DNAs. Thus, the number of probes may be prepared depending on the type of the target DNA. In a case where a genetic mutation is to be detected, a probe having a sequence complementary to the wild-type sequence may be prepared, and the target genetic mutation can also be detected based on a mismatched base, since a melting temperature lower than that of a completely complementary wild-type sequence may be observed when the mismatched base is included. Methods of designing probes are well known in the art, and probes that can be used in the present invention may be designed to have the length and nucleotide composition (melting temperature) which allow specific binding (hybridization). For example, as for the length with a function as a probe, the length of a sequence portion specific to the target DNA may be preferably 10 bases or more, more preferably 15 to 50 bases, and still more preferably 15 to 30 bases, for example, about 20 bases. In designing the probe, it may be preferable to confirm the GC content of the probe and the melting temperature (Tm) of the probe. Known probe design software can be used to confirm Tm.

The probe may be fluorescently labeled and specifically may contain a fluorescent dye, or may contain a fluorescent dye and a quenching dye (quencher). When the probe contains a fluorescent dye, the fluorescence intensity of the fluorescent dye may be used to measure the binding between the DNA and the probe amplified in the amplification reaction to be described later. The fluorescent dye as well as the combination of the fluorescent dye and the quenching dye are well known in the art, and those skilled in the art can select an appropriate one in view of the amplification reaction and melting curve analysis. In one embodiment, 3'-terminal and 5'-terminal sequences of the probe may have complementary sequence portions or interacting structures, and the probe in a free form (when not bound to the amplified DNA) may cause the fluorescent dye to be quenched. Further, in one embodiment, 3'-terminal and 5'-terminal sequences of the probe may have complementary sequence portions, and the 3'-terminal and 5'-terminal sequences of the probe may be bound to form a stem-loop structure. That is, the probe may preferably be a molecular beacon.

In one embodiment, when the probe includes a plurality of types of probes, the probes may be labeled with an identical fluorescent dye.

The designed probe can be chemically synthesized by a known oligonucleotide synthesis method, but may usually be synthesized using a commercially available chemical synthesizer.

The primer set for amplifying the region containing the target DNA may be designed such that the probe described above can bind to a part of the region amplified using the primer set. Such a primer design method is well known in the art, and primers that can be used in the present invention may be designed to satisfy conditions allowing for specific annealing, for example, to have a length and nucleotide composition (melting temperature) that allows specific annealing. For example, the length with a function as a primer may be preferably 10 bases or more, more preferably 15 to 50 bases, and still more preferably 15 to 30 bases, for example, about 20 bases. In designing the primer, it may be preferable to confirm the GC content of the primer and the melting temperature (Tm) of the primer. Known primer design software can be used to confirm Tm. The designed primer can be can be chemically synthesized by a known oligonucleotide synthesis method, but may usually be synthesized using a commercially available chemical synthesizer.

The enzyme may be a polymerase capable of performing an amplification reaction, i.e., an extension reaction using DNA as a template, and any known DNA polymerase can be used. Preferably, the enzyme may be a DNA polymerase used in the amplification reaction performed with a cycle of temperature change.

The additive for preventing DNA secondary structure formation is not particularly limited as long as it has a preventive effect on the DNA secondary structure formation. Examples of the additive for preventing DNA secondary structure formation may include betaine (e.g., trimethylglycine, carnitine, proline betaine, 2-(trimethylammonio)acetate), dimethylsulfoxide (DMSO), formamide, 7-deaza dGTP (7-deaza-2'-deoxyguanosine), and a nonionic surfactant (e.g., Triton X-100, Tween 20 or Nonidet P-40 (NP-40)). Although the mechanism of preventing DNA secondary structure formation varies depending on the type of additive, any additive of any mechanism can be used. For example, a betaine zwitterion prevents DNA secondary structure formation by equally stabilizing both AT and GC base pairs, DMSO and formamide prevent DNA secondary structure formation by reducing the hydrogen bonding between two DNA strands, and 7-deaza dGTP prevents DNA secondary structure formation by reducing the stability of double-stranded DNA (Karunanathie et al., Biochimie 197: 130-143, 2022; A&T Corporation; Internet HP: https://www.aandt.co.jp/jpn/medical/tree/vol_13_2/).

When the reaction solution is prepared by mixing a fluorescent-labeled probe for a target DNA, a primer set for amplifying a region containing the target DNA, a test biological sample, an enzyme, and an additive for preventing DNA secondary structure formation, the amount of each of the components to be mixed may be appropriately set for the amplification reaction to be described later. In one embodiment, the amplification reaction may be performed by increasing the amount of either the forward primer or the reverse primer (asymmetric PCR). For the asymmetric PCR, see, for example, Anal. Chem., 92, 11705-11713, 2020 (NPL 2).

Subsequently, the reaction solution may be divided into a plurality of micro partitions, and an amplification reaction may be performed on each of the plurality of micro partitions. The amplification reaction in micro partitions is known in the art and is referred to, for example, as digital PCR (dPCR) (PTL 1 and NPL 1). Known micro partitions may include wells, droplets, and the like, and are operated such that one molecule of the target DNA is contained or not contained in each of the micro partitions.

The amplification reaction can be performed by any amplification reaction as long as it is an amplification reaction known in the art. In one embodiment, the amplification reaction may be an amplification reaction that is performed with a cycle of temperature change, preferably the polymerase chain reaction (PCR). Preferably, the amplification reaction may be performed by the asymmetric PCR.

Subsequently, the binding between the DNA amplified in the amplification reaction and the probe may be measured. Specifically, the binding between the DNA obtained by the amplification reaction and the probe may be measured by measuring a fluorescence intensity of each of the plurality of micro partitions while changing a temperature of each of the micro partitions. In one embodiment, this step may include measuring a fluorescence intensity while changing a temperature of each of the micro partitions at a sampling interval of less than 1°C, for example at an interval of 0.01°C to 0.99°C, preferably at an interval of 0.3°C to 0.5°C.

Then, based on the measurement result, melting curve analysis may be performed on each of the micro partitions to calculate a melting temperature. Since the binding varies depending on the melting temperature, the melting temperature of the duplex between the amplified DNA and the probe can be calculated from the change in the binding (i.e., the change in the fluorescence intensity) accompanying the temperature change of the solution. Such an operation is referred to as melting curve analysis. Alternatively, the binding between the amplified DNA and the probe may be measured for each cycle of temperature change of the solution, and it may also be possible to determine the target DNA of interest from the relationship between the number of cycles and the change in the binding.

The amplification reaction and the melting curve analysis may be performed as described above, and thus the presence or absence and/or the type of the target DNA may be determined for each micro partition from the fluorescence color (the type of the probe, i.e., the target DNA), the fluorescence intensity (binding), and the melting temperature. In one embodiment, when the target DNA may include a plurality of target genes or target DNAs, melting curve analysis may be performed on each of the plurality of target genes or target DNAs to determine the presence or absence and/or the type of each of the target genes or target DNAs. Further, it may also be possible to quantify the copy number of target genes or target DNAs or the abundance ratio of the target DNA to the wild-type DNA in the test biological sample. In one embodiment, the method may further include a step of counting a number of the target DNA by combining the determination results of the plurality of micro partitions based on the determination.

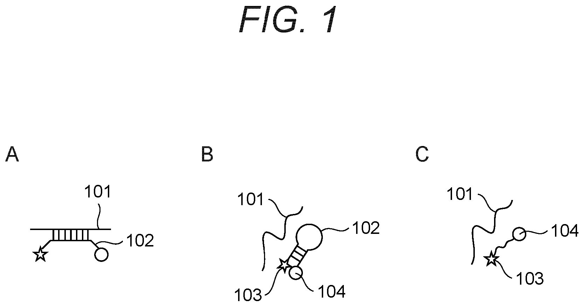

The DNA detection method of the present invention will be specifically described with reference to the schematic view and diagrams of FIGS. 1 to 6.

FIG. 1 is a schematic view showing an example of a method for measuring a melting temperature of DNA using a fluorescent-labeled probe. Examples of the fluorescent-labeled probe may include molecular beacons and Pleiades probes. Here, FIG. 1 will be described using a molecular beacon as an example.

A fluorescent-labeled probe 102, which is a molecular beacon, may be configured as an oligonucleotide, and may have a sequence complementary to a sequence (amplified DNA 101) between a primer pair used in an amplification reaction for amplifying a target DNA. In addition, the molecular beacon may have sequence portions that are complementary to each other at both ends, and a fluorescent dye 103 may be provided at one end of the ends and a quenching dye (quencher) 104 may be provided at the other end. In the amplification reaction, in an initial form (free form), as shown in B of FIG. 1, the fluorescent-labeled probe (molecular beacon) 102 may exist alone in a free form. At that time, the fluorescent-labeled probe (molecular beacon) 102 may form a stem-loop in a complementary sequence portion, the fluorescent dye 103 and the quencher 104 may be close to each other, and thus fluorescence may not be emitted. When a specimen solution is heated in an initial denaturation step, a structure with a high degree of freedom may be formed as in C of FIG. 1. However, since the fluorescent dye and the quenching dye may not be always separated, fluorescence remains quenched. In an annealing step, when the temperature is lowered to a temperature of about room temperature, a loop portion of the fluorescent-labeled probe (molecular beacon) 102 may be annealed to DNA 101 amplified in the specimen solution as shown in A of FIG. 1. As a result, the fluorescent dye 103 and the quencher 104 may always be separated from each other, and thus the fluorescent-labeled probe 102 may emit strong fluorescence. In the next extension step, the fluorescent-labeled probe (molecular beacon) 102 may be dissociated as shown in B of FIG. 1 again, and the fluorescence may be quenched. In the next denaturation step, as shown in C of FIG. 1 again, the fluorescence may remain quenched. Since this step is repeated in the amplification reaction, the fluorescence intensity may be measured at any stage during heating or cooling. When the fluorescence intensity is measured after completion of the amplification reaction, the fluorescence intensity can be measured in a similar manner. After completion of the amplification reaction, heating or cooling may be performed only to measure the fluorescence intensity, and then the fluorescence intensity may be measured. At this time, a change in the fluorescence intensity accompanying the temperature change may be plotted to create a melting curve, and the melting temperature can be calculated from the melting curve.

In the fluorescent-labeled probe (molecular beacon) 102 used here, the combination of the fluorescent dye 103 and the quencher 104 may not be particularly limited as long as it is a combination generally used for real-time PCR. Examples of the fluorescent dye 103 may include FAM, VIC, ROX, Cy3, and Cy5, and examples of the quencher 104 may include TAMRA, BHQ1, BHQ2, and BHQ3. All of them may be commonly used and may be available as commercial products.

When two types having different sequences are targeted as genes to be analyzed, the two types of target genes or target DNAs can be distinguished and detected in one reaction system by preparing sequences of the fluorescent-labeled probe (molecular beacon) 102 that specifically bind to the target genes or target DNAs, and binding different fluorescent dyes thereto. Alternatively, the sequences of the molecular beacon may be designed such that the melting temperatures of two types of molecular beacons are different, and thus, even when an identical fluorescent dye is bound, the two types of target genes or target DNAs can be distinguished and detected in one reaction system.

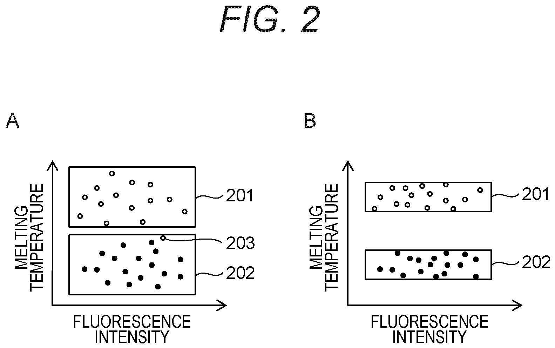

FIG. 2 shows an example of a result obtained by a method for determining and counting the presence or absence of a plurality of genes in each micro partition based on the melting temperature measured using a fluorescent-labeled probe in digital PCR (dPCR). Similarly to A of FIG. 1, when the variation in melting temperature is large, melting temperature distributions of two types of genes, i.e., a melting temperature distribution 201 of a gene having a high melting temperature and a melting temperature distribution 202 of a gene having a low melting temperature, may be close to each other with a spread, and a micro partition 203 containing a gene having a high melting temperature may erroneously be observed within the threshold of the melting temperature distribution 202 of a gene having a low melting temperature, and may be counted as a different gene. Meanwhile, in a case where the variation in melting temperature is small as in B of FIG. 1, even when the average value of the melting temperature distributions of the two types of genes is the same as that in A of FIG. 1, the melting temperature distributions 201 and 202 may be separated, and the probability of erroneous determination of the type of gene may be reduced.

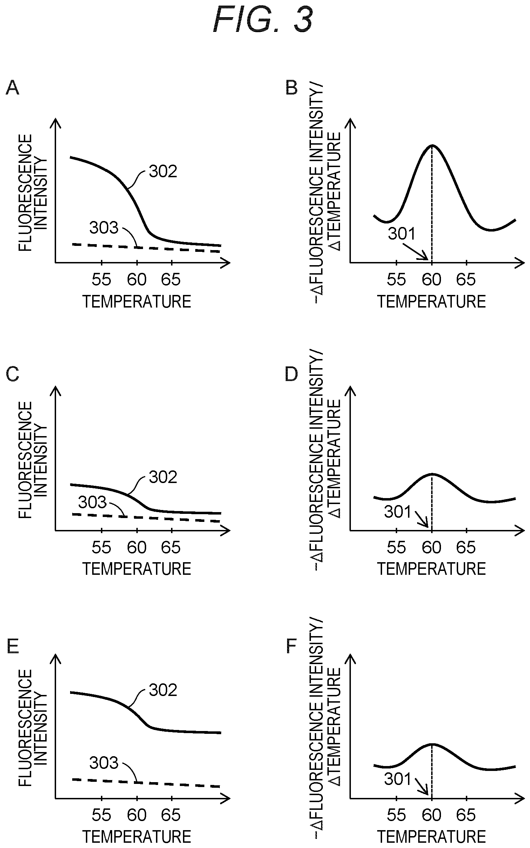

FIG. 3 shows graphs showing an example of melting curves of a micro partition containing a gene having a large or small variation in melting temperature. A of FIG. 3 shows an example of a melting curve of a gene having a small variation in melting temperature, and C and D of FIG. 3 show examples of a melting curve of a gene having a large variation in melting temperature. As shown in A of FIG. 3, in a melting curve 302 of the micro partition (positive well) containing the target DNA, the fluorescent-labeled probe may bind to the corresponding DNA and may exhibit high fluorescence intensity at low temperature, and the fluorescent-labeled probe may be dissociated from the corresponding DNA with an increase in temperature, and thus the fluorescence intensity may decrease. A melting curve 303 of the micro partition (negative well) not containing the target DNA may have no change in the fluorescence intensity accompanying the temperature change, because the micro partition does not contain DNA to which the fluorescent-labeled probe binds. When a differential curve is determined from A of FIG. 3, a high peak is shown at 60°C as in B of FIG. 3, and the temperature of the peak is a melting temperature (Tm) 301. Meanwhile, an example of a melting curve when a gene having a large variation in melting temperature is measured is shown in C of FIG. 3, and a differential curve thereof is shown in D of FIG. 3. As shown in C of FIG. 3, in the melting curve 302 of the micro partition containing the target DNA, the fluorescent-labeled probe may bind to the corresponding DNA at low temperature, but the binding amount may be small, and the fluorescence intensity may be lower than that in A of FIG. 3. As a reaction occurring at this time, one strand of DNA excessively amplified by the asymmetric PCR may form a secondary structure, and the fluorescent-labeled probe may be less likely to bind, and thus the binding amount of the fluorescent-labeled probe may be reduced. Accordingly, as shown in D of FIG. 3, the differential curve may have a low peak height and may become a broad peak (a peak having a shape that extends laterally and has a low height). Another example of a melting curve when a gene having a large variation in melting temperature is measured is shown in E of FIG. 3, and a differential curve thereof is shown in F of FIG. 3. As shown in E of FIG. 3, in the melting curve 302 of the micro partition containing the target DNA, the fluorescent-labeled probe may bind to the corresponding DNA and may exhibit high fluorescence intensity at low temperature. However, even when the fluorescent-labeled probe is dissociated from the corresponding DNA with an increase in temperature, the decrease in fluorescence intensity may be small, and the change in the fluorescence intensity accompanying the temperature change may be smaller than that in A of FIG. 3. As a reaction occurring at this time, the fluorescent-labeled probe may be dissociated from the corresponding DNA, and then the fluorescent-labeled probe may form a secondary structure different from the stem-loop, and the fluorescent dye and the quenching dye may not quench without forming a close structure, and may emit fluorescence. Accordingly, as shown in F of FIG. 3, the differential curve has a low peak height and becomes a broad peak. C and E of FIG. 3 may have different shapes of melting curves, but may have a common feature that the change in the fluorescence intensity accompanying the temperature change is small. As a result, differential curves of the melting curves may both have broad peaks as in D and F of FIG. 3. In the differential curve having a broad peak, the variation of melting temperature 301 may easily increase due to measurement errors.

According to the present invention, when an additive for preventing DNA secondary structure formation is added to the reaction solution, the melting curve may change from the shapes of C and E in FIG. 3 to the shape of A in FIG. 3. Further, the differential curve of the melting curve which is a broad peak may also change to a sharp peak as in B of FIG. 3. As a result, the variation in the melting temperature 301 due to measurement errors may be reduced.

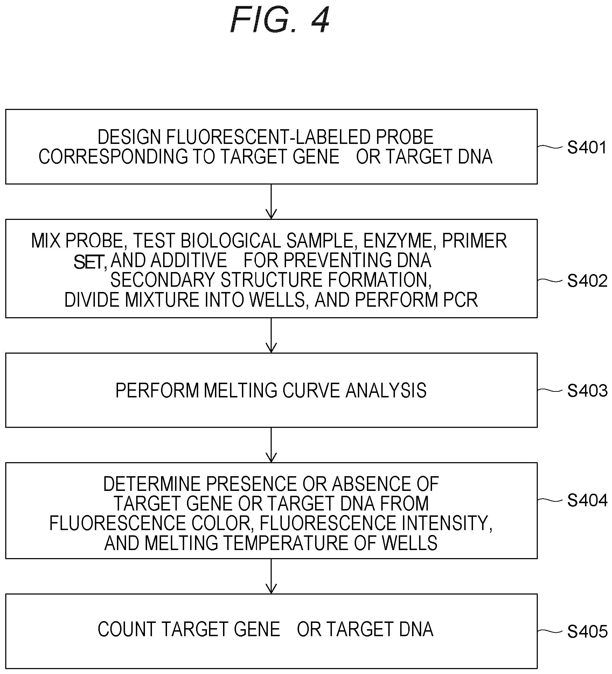

FIG. 4 is a flowchart showing one embodiment of a DNA detection method including: adding an additive for preventing DNA secondary structure formation to a reaction solution; performing digital PCR using melting curve analysis; and quantifying the copy number of target DNA. First, probes corresponding to a plurality of target genes or target DNAs are designed (S401). A probe, a biological sample, an enzyme, a primer set, and an additive for preventing DNA secondary structure formation may be mixed, the mixture may be divided into wells, and then PCR may be performed in each of the wells (S402). A melting curve between the probe corresponding to each of the target genes or target DNAs and the amplified product (amplified DNA) may be obtained by melting curve analysis (S403). The presence or absence of the target DNA may be determined from the fluorescence color, fluorescence intensity, and melting temperature of each of the wells (S404). Finally, wells containing each of the target genes or target DNAs and wells not containing both target genes or target DNAs may be counted, and their starting concentrations may be calculated by Poisson correction (S405).

When there is a plurality of target genes or target DNAs to be detected, a plurality of primers and a plurality of probes may be prepared in accordance with the target genes or target DNAs. In addition, an amplification reaction (e.g., asymmetric PCR) may be performed on a test biological sample containing or possibly containing DNA of the plurality of target genes or target DNAs. The plurality of probes may be designed by changing the melting temperature of DNA of the target genes or target DNAs or changing the type of fluorescent dye, and thus it may be possible to discriminate the type of target genes or target DNAs contained in the solution from the fluorescence color and melting temperature of the solution after the amplification reaction.

In the DNA detection method according to the present invention, when a target DNA may be detected by a combination of PCR and melting curve analysis, PCR and melting curve analysis may be performed in the presence of the additive for preventing DNA secondary structure formation, or melting curve analysis may be performed in the presence of the additive for preventing DNA secondary structure formation, whereby a variation in melting temperature may be reduced and an error bar may also be reduced. Thus, the target DNA, particularly a plurality of target genes or target DNAs, can be detected or quantified with accuracy and high sensitivity.

### (2) DNA detection kit

The above-described method of the present invention can be more easily and simply performed by using a kit including at least an additive for preventing DNA secondary structure formation.

That is, in one aspect, the present invention provides a DNA detection kit, and the kit includes:
a primer pair for amplifying a region including a target DNA, the primer pair including a forward primer and a reverse primer;
a fluorescent-labeled probe for the target DNA, which binds to DNA amplified using the forward primer and the reverse primer; and
an additive for preventing DNA secondary structure formation.

The fluorescent-labeled probe may be as described in the previous section, and the fluorescent dye or the melting temperature may vary for each target DNA. In one embodiment, the probe may contain a fluorescent dye and a quenching dye, 3'-terminal and 5'-terminal sequences of the probe may have complementary sequence portions or interacting structures, and the probe in a free form may cause the fluorescent dye to be quenched.

The forward primer and the reverse primer may be as described in the previous section. In one embodiment, concentrations of the forward primer and the reverse primer included in the kit of the present invention may be different, and the concentration of either the forward primer or the reverse primer may be made higher (for the asymmetric PCR). Desirably, the concentration of the primer that synthesizes the complementary strand of the fluorescent-labeled probe may be higher.

The additive for preventing DNA secondary structure formation may be as described in the previous section. In one embodiment, the additive for preventing DNA secondary structure formation may include at least one selected from the group consisting of betaine (e.g., trimethylglycine), dimethylsulfoxide (DMSO), formamide, 7-deaza dGTP, and a nonionic surfactant.

The kit according to the present invention may further include a DNA polymerase. The DNA polymerase is also as described above and any DNA polymerase can be used.

The kit according to the present invention may further include other components necessary for performing the amplification reaction, for example, a substrate. Further, the kit may include instructions describing the procedure and protocol for determining the target DNA.

In one embodiment, the DNA detection kit of the present invention can be used for performing the DNA detection method of the present invention described above. Alternatively, the DNA detection kit of the present invention can be used for detecting a target DNA by a combination of PCR and melting curve analysis, and an additive for preventing DNA secondary structure formation may be added when PCR and melting curve analysis are performed, or an additive for preventing DNA secondary structure formation may be added when melting curve analysis is performed. The DNA detection kit of the present invention may be useful for determining the presence or absence of the target DNA (particularly a plurality of target genes or target DNAs) and determining the positional relationship (cis form or trans form) of these target genes or target DNAs as alleles.

In addition, the DNA detection kit of the present invention can be used for evaluating the phenotype (e.g., the possibility of affection of a disease or disorder or drug responsiveness) associated with the target DNA (particularly, genetic mutation). Examples

### [Example 1]

In this example, measurement results in a case where digital PCR using melting curve analysis is performed without adding an additive for preventing DNA secondary structure formation will be described.

Wild-type genomic DNA (final concentration: 133 molecules/µL, genomic DNA reference standard (KRAS Wild Type Reference Standard, Catalog ID: HD710, manufactured by Horizon Discovery Ltd.) was prepared. A forward primer corresponding to each target DNA required for PCR (final concentration: 0.25 µM), a reverse primer corresponding to the target DNA (final concentration: 2.0 µM), a fluorescent-labeled probe corresponding to the target DNA (final concentration: 0.5 µM), and 1x master mix (including DNA polymerase and dNTP) were added to prepare a PCR reaction solution. At this time, the primer pair was added such that the concentration of the primer pair was asymmetric such that the complementary DNA strand of the fluorescent-labeled probe was excessively amplified (the concentration of the reverse primer was set high). Sequences of primers and probes are as follows.
Forward primer corresponding to KRAS gene: 5'-GTCACATTTTCATTATTTTTATTATAAGG-3' (SEQ ID NO: 1)
Reverse primer corresponding to KRAS gene: 5'-GTCAAGGCACTCTTGCCTAC-3' (SEQ ID NO: 2)
Fluorescent-labeled probe corresponding to KRAS gene: 5'-CTCGATGTGAGTTTCTGC-3' (SEQ ID NO: 3)
Forward primer corresponding to hTERT gene: 5'-GGGTCCTCGCCTGTGTACAG-3' (SEQ ID NO: 4)
Reverse primer corresponding to hTERT gene: 5'-CCTGGGAGCTCTGGGAATTT-3' (SEQ ID NO: 5)
Fluorescent-labeled probe corresponding to hTERT gene: 5'-CACACCTTTGGTCACTC-3' (SEQ ID NO: 6)
Forward primer corresponding to GNAS gene: 5'-GCTTTGGTGAGATCCATTGAC-3' (SEQ ID NO: 7)
Reverse primer corresponding to GNAS gene: 5'-TCCACCTGGAACTTGGTCTC-3' (SEQ ID NO: 8)
Fluorescent-labeled probe corresponding to GNAS gene: 5'-TTCGCTGCCGTGTCC-3' (SEQ ID NO: 9)

Note that as all of the fluorescent-labeled probes, EasyBeacons (PentaBase A/S) with a specific hydrophobic base HyNA (TM) near both ends were used. In the case of free fluorescent-labeled probes, they are designed to form a hydrophobic bond in the molecule. Further, in each of the fluorescent-labeled probes, HEX as a fluorescent dye is bound to the 5' end, and BHQ-1 as a quencher is bound to the 3' end.

Thereafter, 15 µL of the PCR reaction solution was divided into 20,000 wells, and DNA was amplified by PCR. In the PCR reaction, reaction was performed at 96°C for 10 minutes, followed by 59 cycles of cycles (60°C for 2 minutes, then 98°C for 30 seconds), and finally at 60°C for 2 minutes. After the reaction, while a chip provided with the well was heated from 45°C to 80°C on a temperature control stage, a fluorescence image of the chip was acquired at an interval of 0.35°C by a digital PCR apparatus for melting curve analysis, a change in the fluorescence intensity of each well was observed from the obtained fluorescence image, and melting curves were measured and analyzed.

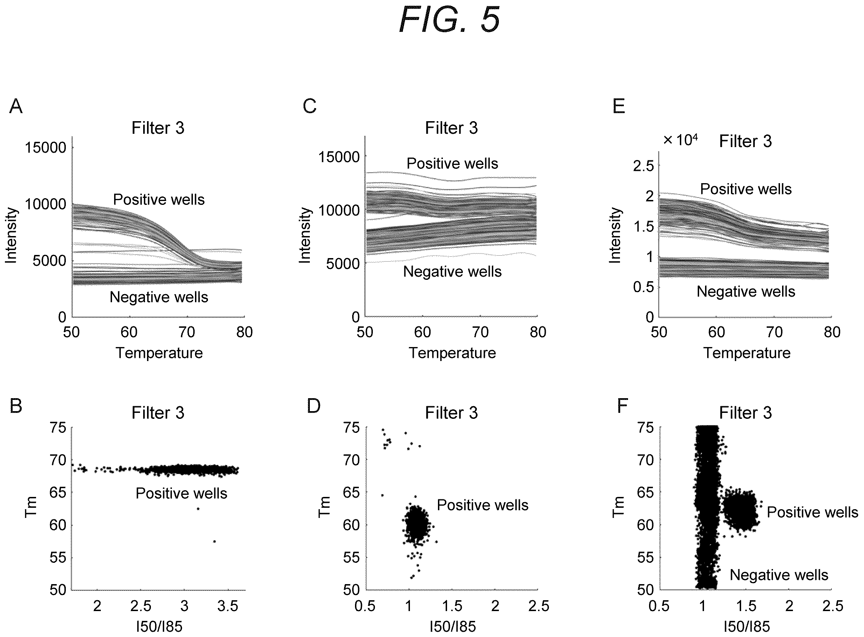

FIG. 5 shows results of measuring the melting curve and the distribution of the melting temperature of each gene when an additive for preventing DNA secondary structure formation is not added. A and B of FIG. 5 show the distribution of the melting curve and melting temperature of the KRAS gene, C and D of FIG. 5 show the distribution of the melting curve and melting temperature of the hTERT gene, and E and F of FIG. 5 show the distribution of the melting curve and melting temperature of the GNAS gene. Referring to A of FIG. 5, the melting curve of the KRAS gene showed a large change in the fluorescence intensity accompanying the temperature change. As shown in B of FIG. 5, the melting temperature had a small variation, and the variation was 0.33°C or less. Meanwhile, referring to C of FIG. 5, the melting curve of the hTERT gene showed a small change in the fluorescence intensity accompanying the temperature change. As shown in D of FIG. 5, the melting temperature had a large variation, and the variation was 0.90°C. Furthermore, referring to E of FIG. 5, in the melting curve of the GNAS gene, a difference in fluorescence intensity between the positive well containing the target DNA and the negative well not containing the target DNA was large at low temperature. However, a decrease in fluorescence intensity of the positive well due to an increase in temperature was small, and the fluorescence intensity remained high even at high temperature. As a result, a change in the fluorescence intensity accompanying the temperature change was small, the variation in the melting temperature was large as shown in F of FIG. 5, and the variation was 0.97°C.

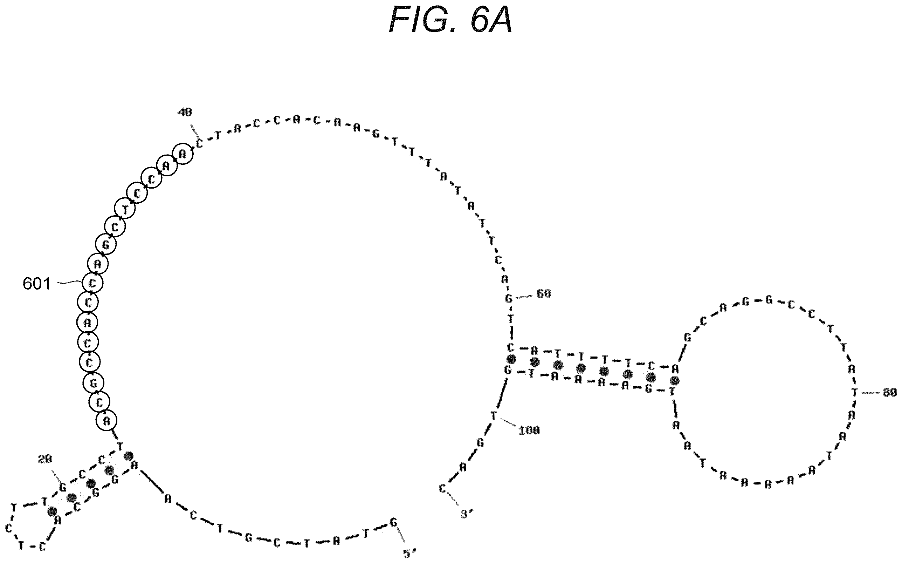

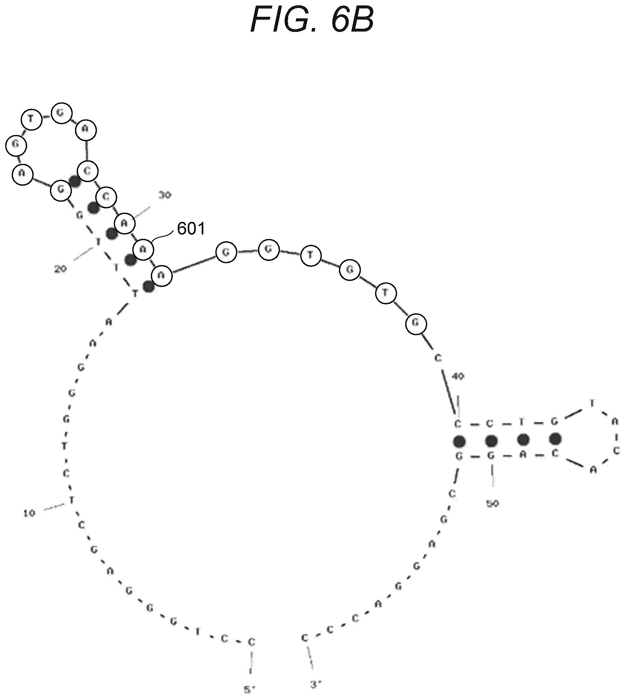

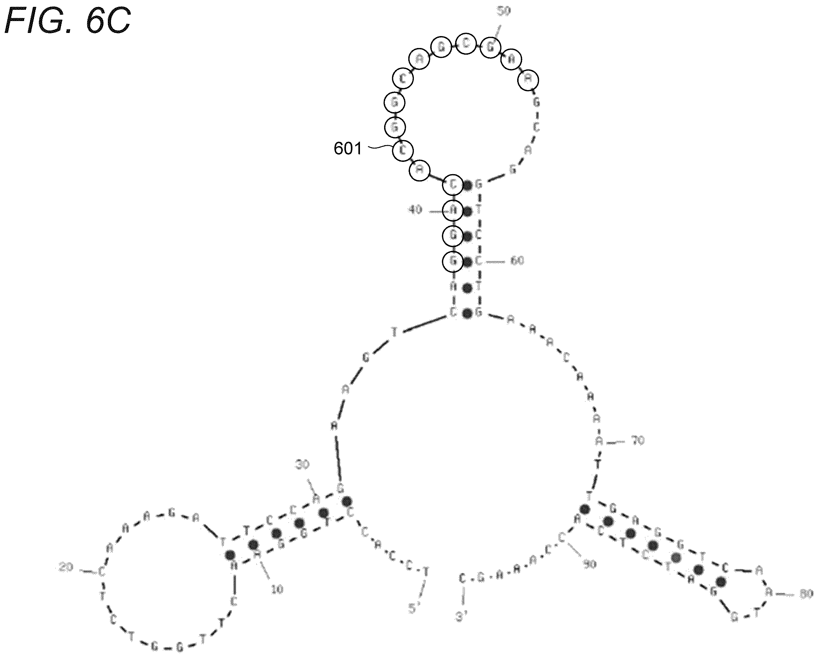

There will be examined sequences of amplicons and fluorescent-labeled probes of KRAS gene having a small variation in melting temperature and hTERT and GNAS genes having a large variation in melting temperature. FIGS. 6A, 6B, and 6C show the sequences (SEQ ID NOs: 10 to 12) of amplicons corresponding to KRAS, hTERT, and GNAS genes, respectively, along with predicted secondary structures and probe binding regions 601. Amplicons may form a double-stranded structure in themselves, as shown in FIGS. 6A to 6C. In FIGS. 6A to 6C, each region 601 to which the fluorescent-labeled probe binds is circled. FIG. 6A shows that, in the KRAS gene having a small variation in melting temperature, a ratio at which a fluorescent-labeled probe binding region of one strand of DNA excessively amplified by the asymmetric PCR forms a double-stranded structure is small. Meanwhile, referring to the sequences of the amplicons and the fluorescent-labeled probes of the hTERT and GNAS genes having a large variation in melting temperature, as shown in FIGS. 6B and 6C, the ratio at which the fluorescent-labeled probe binding region of one strand of DNA excessively amplified by the asymmetric PCR forms a double-stranded structure is large. Furthermore, it is found that when the fluorescent-labeled probe is redesigned such that the fluorescent-labeled probe binding region of the KRAS gene is changed from a region in which the ratio of forming a double-stranded structure of one strand of DNA excessively amplified is low to a region in which the ratio described above is high, the fluorescence intensity at low temperature is reduced (PCT/JP2022/024591).

These results show that the DNA secondary structure formation may contribute to an increase in variation in melting temperature.

### [Example 2]

From the results of Example 1, it was expected that the variation in melting temperature would be reduced by preventing the DNA secondary structure formation. Therefore, in this example, an example is shown in which the variation in melting temperature is reduced and the target DNA is accurately measured by adding an additive for preventing DNA secondary structure formation and performing real-time PCR using melting curve analysis.

Similarly to Example 1, wild-type genomic DNA (final concentration: 133 molecules/µL) was prepared. A forward primer corresponding to the hTERT gene required for PCR (final concentration: 0.25 µM), a reverse primer corresponding to the hTERT gene (final concentration: 2.0 µM), a fluorescent-labeled probe corresponding to the hTERT gene (final concentration: 0.5 µM), and 1x master mix (including DNA polymerase and dNTP) were added. To this PCR reaction solution, the additive: trimethylglycine, DMSO, or formamide, i.e., a type of betaine, was added at different concentrations. At this time, the primer pair was added such that the concentration of the primer pair was asymmetric such that the complementary DNA strand of the fluorescent-labeled probe was excessively amplified (the concentration of the reverse primer was set high). Primers and probes are identical to the primers and probes used in Example 1.

In the PCR reaction, reaction was performed at 95°C for 20 minutes, followed by 60 cycles of cycles (95°C for 1 second, then 60°C for 20 seconds). After the reaction, a change in fluorescence intensity was observed with a real-time PCR apparatus while heating from 50°C to 95°C, and a melting curve was measured and analyzed.

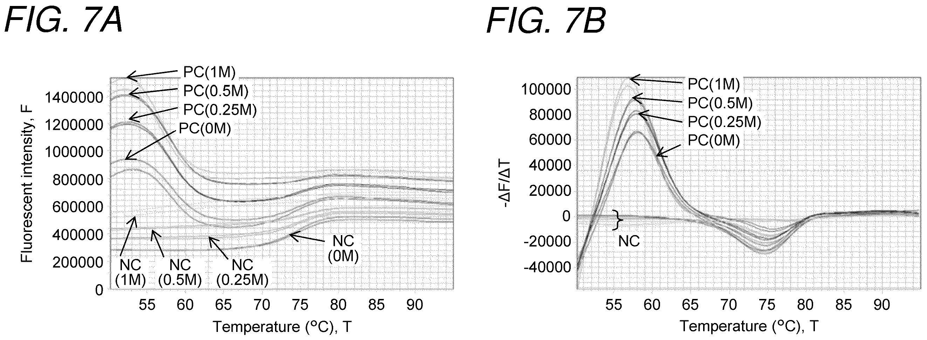

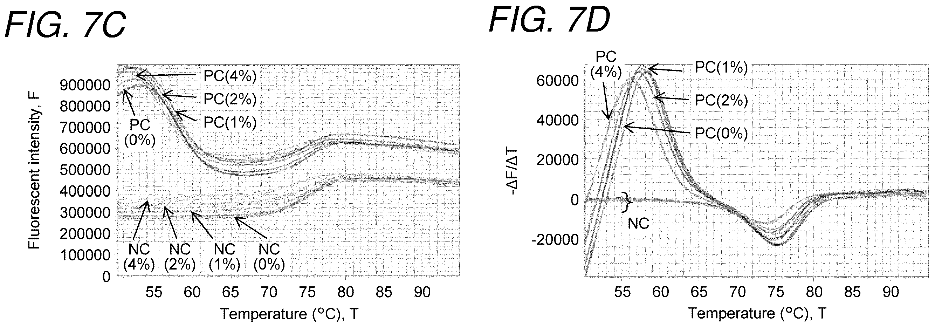

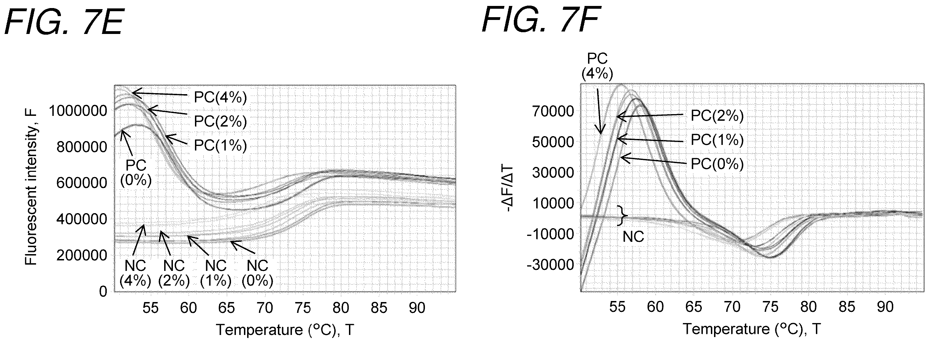

FIG. 7A shows melting curves when betaine (trimethylglycine) was added at a concentration varying from 0 M to 1 M, and FIG. 7B shows differential curves of the melting curves. FIG. 7C shows melting curves when DMSO was added at a concentration varying from 0% to 4%, and FIG. 7D shows differential curves of the melting curves. FIG. 7E shows melting curves when formamide was added at a concentration varying from 0% to 4%, and FIG. 7F shows differential curves of the melting curves. In FIGS. 7A to 7F, PC represents a melting curve of a well containing a target DNA and a differential curve thereof, and NC represents a melting curve of a well not containing a target DNA and a differential curve thereof. Each numerical value in parentheses after PC and NC represents the concentration of each additive.

Even in a case where any additive was used, the shape of the melting curve changed in the presence of the additive, and the higher the concentration of the additive was, the higher the fluorescence intensity at low temperature was (FIGS. 7A to 7F). As a result, when the differential curve was observed, the higher the concentration of the additive was, the higher the peak value of the differential curve was. In the case of the addition of trimethylglycine among the three types of additives, the shape change of the melting curve and its differential curve was the largest.

### [Example 3]

In this example, trimethylglycine, i.e., a type of betaine, was added to the PCR reaction solution, and digital PCR using melting curve analysis was performed to evaluate the variation in melting temperature.

Similarly to Example 1, wild-type genomic DNA (final concentration: 133 molecules/µL) was prepared. A forward primer corresponding to each target DNA required for PCR (final concentration: 0.25 µM), a reverse primer corresponding to the target DNA (final concentration: 2.0 µM), a fluorescent-labeled probe corresponding to the target DNA (final concentration: 0.5 µM), 0.5M trimethylglycine, and 1x master mix (including DNA polymerase and dNTP) were added to prepare a PCR reaction solution. At this time, the primer pair was added such that the concentration of the primer pair was asymmetric such that the complementary DNA strand of the fluorescent-labeled probe was excessively amplified (the concentration of the reverse primer was set high). Primers and probes are identical to the primers and probes used in Example 1.

Thereafter, 15 µL of the PCR reaction solution was divided into 20,000 wells, and DNA was amplified by PCR. In the PCR reaction, reaction was performed at 96°C for 10 minutes, followed by 59 cycles of cycles (60°C for 2 minutes, then 98°C for 30 seconds), and finally at 60°C for 2 minutes. After the reaction, while a chip provided with the well was heated from 45°C to 80°C on a temperature control stage, a fluorescence image of the chip was acquired at an interval of 0.35°C by a digital PCR apparatus for melting curve analysis, a change in the fluorescence intensity of each well was observed from the obtained fluorescence image, and melting curves were measured and analyzed.

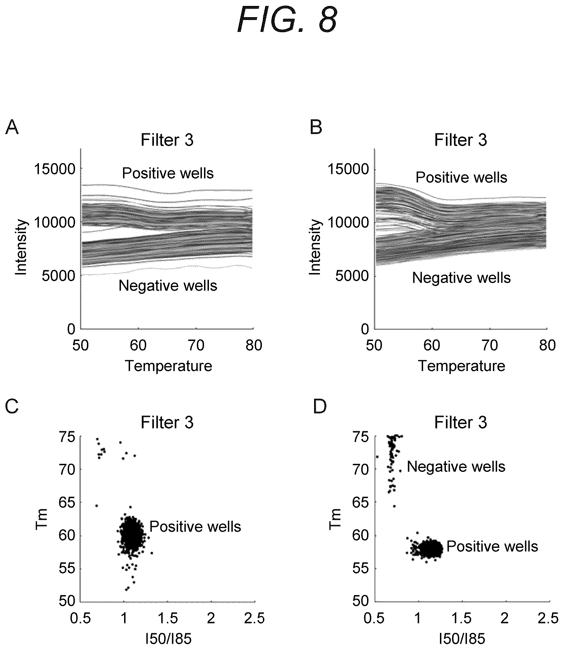

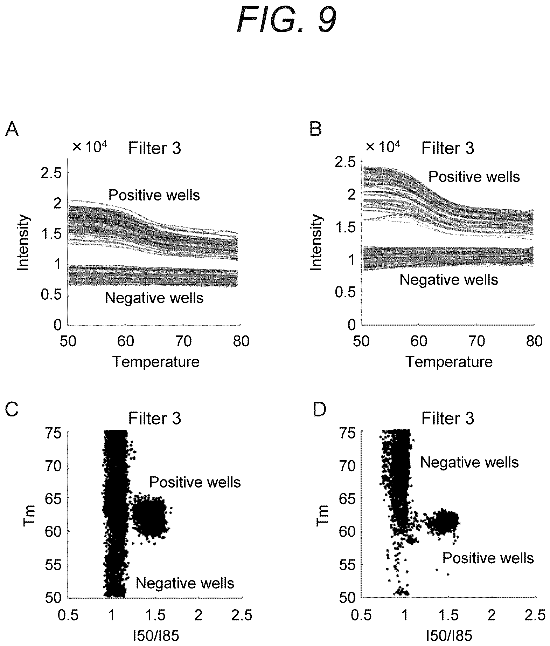

FIG. 8 shows the results of performing digital PCR using melting curve analysis in the presence or absence of trimethylglycine to measure variations in the melting curve and melting temperature of the hTERT gene. Similarly, FIG. 9 shows the results of measuring the melting curve of GNAS gene and the variation in melting temperature. In FIGS. 8 and 9, A shows melting curves in the absence of trimethylglycine, B shows melting curves in the presence of trimethylglycine, C shows a distribution of melting temperature in the absence of trimethylglycine, and D shows a distribution of melting temperature in the presence of trimethylglycine. It can be seen that the variation in melting temperature is reduced in both the hTERT gene and the GNAS gene.

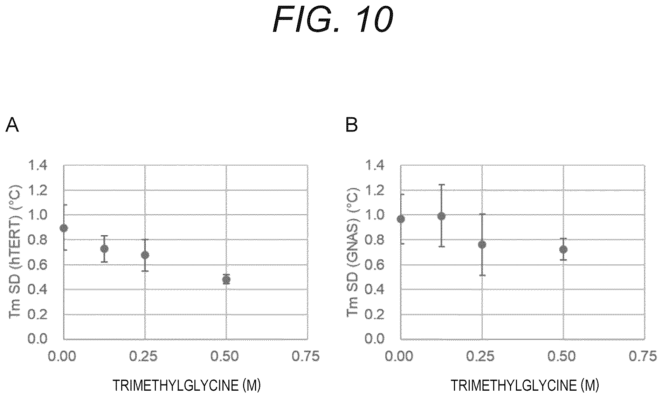

A and B of FIG. 10 show graphs summarizing variations in trimethylglycine concentration and melting temperature for hTERT gene and GNAS gene. In both the hTERT gene and the GNAS gene, the higher the concentration of trimethylglycine, the smaller the distribution of melting temperature. As a result of the addition of 0.5 M trimethylglycine, the distribution of hTERT gene melting temperature was reduced from 0.90°C to 0.48°C (47% reduction) (A in FIG. 10) and the distribution of GNAS gene Tm value was reduced from 0.97°C to 0.72°C (26% reduction) (B in FIG. 10). Further, variations between measurements in a plurality of measurements were indicated by error bars in A and B of FIG. 10. The error bar in each graph was reduced as the concentration of trimethylglycine was increased, which suggested that the addition of betaine such as trimethylglycine reduced the difference between the measurements of the distribution of melting temperature, and increased the reproducibility of the measurement results.

As described above, an additive for preventing DNA secondary structure formation may be added and digital PCR using melting curve analysis is performed, as a result of which the variation in melting temperature is reduced, and the target DNA can be accurately identified based on the melting temperature, the fluorescent dye color, and the fluorescence intensity.

### Reference Signs List

- 101: DNA
- 102: fluorescent-labeled probe
- 103: fluorescent dye
- 104: quencher
- 201: melting temperature distribution of gene having high melting temperature
- 202: melting temperature distribution of gene having low melting temperature
- 203: micro partition containing gene having high melting temperature
- 301: melting temperature
- 302: melting curve of micro partition containing target DNA
- 303: melting curve of micro partition not containing target DNA
- 601: fluorescent-labeled probe binding region

### Sequence Listing Free Text

SEQ ID NOs: 1 to 12: DNA (synthetic construct), synthetic polynucleotide

## Claims

1. A DNA detection method comprising the steps of:
mixing a fluorescent-labeled probe for a target DNA, a primer set for amplifying a region containing the target DNA, a test biological sample, an enzyme, and an additive for preventing DNA secondary structure formation to prepare a reaction solution;
dividing the reaction solution into a plurality of micro partitions and performing an amplification reaction in each of the plurality of micro partitions;
measuring a fluorescence intensity for each of the plurality of micro partitions while changing a temperature and measuring binding between the DNA amplified in the amplification reaction and the probe;
performing melting curve analysis on each of the micro partitions based on the measurement result to calculate a melting temperature; and
determining a presence or absence and/or a type of the target DNA from a fluorescence color, fluorescence intensity, and melting temperature of each of the micro partitions.

2. The method according to claim 1, wherein
the probe comprises a fluorescent dye and a quenching dye, and
a fluorescence intensity of the fluorescent dye is used to measure binding between the amplified DNA and the probe.

3. The method according to at least one of the previous claims, wherein 3'-terminal and 5'-terminal sequences of the probe have complementary sequence portions or interacting structures, and the probe in a free form causes the fluorescent dye to be quenched.

4. The method according to at least one of the previous claims, further comprising a step of counting a number of the target DNA by combining the determination results of the plurality of micro partitions based on the determination.

5. The method according to at least one of the previous claims, wherein the step of measuring a fluorescence intensity while changing a temperature of each of the micro partitions comprises measuring a fluorescence intensity while a temperature is changed at a sampling interval of less than 1°C.

6. The method according to at least one of the previous claims, wherein
the target DNA comprises a plurality of target genes or target DNAs, and
melting curve analysis is performed on each of the plurality of target genes or target DNAs to determine the presence or absence and/or the type of each of the target genes or target DNAs.

7. The method according to at least one of the previous claims, wherein the additive for preventing DNA secondary structure formation comprises at least one selected from the group consisting of betaine, dimethylsulfoxide (DMSO), formamide, 7-deaza dGTP, and a nonionic surfactant.

8. The method according to at least one of the previous claims, wherein the step of performing the amplification reaction is performed by an asymmetric PCR.

9. The method according to at least one of the previous claims, wherein the amplification reaction is performed with a cycle of temperature change.

10. The method according to at least one of the previous claims, wherein when the probe comprises a plurality of types of probes, the probes are labeled with an identical fluorescent dye.

11. A DNA detection kit comprising:
a primer pair for amplifying a region comprising a target DNA, the primer pair comprising a forward primer and a reverse primer;
a fluorescent-labeled probe for the target DNA, which binds to DNA amplified using the forward primer and the reverse primer; and
an additive for preventing DNA secondary structure formation.

12. The kit according to claim 11, further comprising a DNA polymerase.

13. The kit according to at least one of the claims 11 to 12, wherein the additive for preventing DNA secondary structure formation comprises at least one selected from the group consisting of betaine, dimethylsulfoxide (DMSO), formamide, 7-deaza dGTP, and a nonionic surfactant.

14. The kit according to at least one of the claims 11 to 13, for use in performing the method according to claim 1.

15. The kit according to at least one of the claims 11 to 14, wherein
the probe comprises a fluorescent dye and a quenching dye,
3'-terminal and 5'-terminal sequences of the probe have complementary sequence portions or interacting structures, and the probe in a free form causes the fluorescent dye to be quenched.
